# EUROPÄISCHE PATENTSCHRIFT

(11) **EP 1 186 609 B1**
(45) Veröffentlichungstag und Bekanntmachung des Hinweises auf die Patenterteilung: **03.12.2008**
(21) Anmeldenummer: 01119799.3
(22) Anmeldetag: 29.08.2001
(51) Int. Cl.: C07F 9/50, C07D 321/10, C07C 43/23, C07F 9/655, C07F 9/572, C07C 309/63, C07F 15/00, B01J 31/24

(54) **Diphosphine**
Diphosphines
Diphosphines

(30) Priorität: 11.09.2000 DE 10044793
(43) Veröffentlichungstag der Anmeldung: 13.03.2002
(73) Patentinhaber: Saltigo GmbH, 40764 Langenfeld (DE)
(72) Erfinder: Driessen-Hölscher, Birgit, Dr., 52074 Aachen (DE); Kralik, Joachim, 64289 Darmstadt (DE); Ritzkopf, Inga c/o Max-Planck-Institut f. Kohlenst, 45466 Mülheim/Ruhr (DE); Steffens, Christian, 52074 Aachen (DE); Giffels, Guido, Dr., 53177 Bonn (DE); Dreisbach, Claus, Dr., 51065 Köln (DE); Prinz, Thomas, Dr., 51371 Leverkusen (DE); Lange, Walter, Dr., 51519 Odenthal (DE)
(74) Vertreter: Wichmann, Birgid

(56) Entgegenhaltungen:
- DE-A- 19 522 293
- US-A- 5 399 771
- DELOGU G ET AL: "Two new efficient preparations of enantiopure 2,2'- dihydroxy-6,6'-dimethoxy-1,1'-biphenyl" TETRAHEDRON: ASYMMETRY, ELSEVIER SCIENCE PUBLISHERS, AMSTERDAM, NL, Bd. 8, Nr. 5, 13. März 1997 (1997-03-13), Seiten 759-763, XP004094336 ISSN: 0957-4166
- DELOGU G ET AL: "Preparation and resolution of 2,2'-dimercapto-6,6'- dimethoxy-1,1'-biphenyl: a C2-symmetric sulfur building block" TETRAHEDRON: ASYMMETRY, ELSEVIER SCIENCE PUBLISHERS, AMSTERDAM, NL, Bd. 9, Nr. 16, 1. August 1998 (1998-08-01), Seiten 2819-2826, XP004134456 ISSN: 0957-4166
- TAKANORI HIGASHIZIMA ET AL: "SYNTHESIS OF NEW CHIRAL PHOSPHINEPHOSPHITES HAVING 2-DIPHENYLPHOSPHINO-BIPHENYL-2'-YL BACKBONE AND THEIR USE IN RH(I)-CATALYZED ASYMMETRIC HYDROFORMYLATIONS" TETRAHEDRON LETTERS, ELSEVIER SCIENCE PUBLISHERS, AMSTERDAM, NL, Bd. 35, Nr. 13, 28. März 1994 (1994-03-28), Seiten 2023-2026, XP000434280 ISSN: 0040-4039

## Beschreibung

Die vorliegende Erfindung betrifft ein Verfahren zur Herstellung racemischer Diphosphine, ein Verfahren zur Herstellung enantiomerenreiner Diphosphine, neue enantiomerenreine Disphosphine, neue Zwischenprodukte zur Herstellung von Diphosphinen und Katalysatoren, die neue Diphosphine enthalten.

Vom erfindungsgemäßen Verfahren zur Herstellung von Diphosphinen stark abweichende Verfahren sind aus der EP-A 749 973 und der DE 195 22 293 A1, bekannt. Soweit man danach enantiomerenreine Diphosphine herstellen will, erfolgt die Racematspaltung auf der Stufe der Phosphinoxide, d.h. für einzelne Diphosphine müssen jeweils separate Racematspaltungen durchgeführt werden. Von den erfindungsgemäßen Stoffen unterschiedliche Verbindungen sind beschrieben in EP-A 104 375, EP-A 582 692, und EP-A 690 065. Racematspaltungen mit N-Benzylcinchonidiniumchlorid sind bisher nur für Dinaphtholverbindungen beschrieben worden (Tetrahedron Lett. 36, 7991 (1995)). Zudem existieren Verfahren zur Herstellung von enantiomerreinen Biphenylenverbindungen über verschiedene Zwischenprodukte wie Phosphorthioamidate (Tetrahedron: Asymmetry. 8, 759 - 763 (1997); Tetrahedron: Asymmetry. 9, 2819 - 2826 (1998)) und chiralen Phosphindiphosphiten (Tetrahedron Lett. 35, 2023 - 2026, (1994). Ein Verfahren zur Herstellung von chiralen Binaphthylderivaten ist ebenfalls beschrieben worden (US-A-5 399 771).

Die vorliegende Erfindung betrifft im Einzelnen zunächst ein Verfahren zur Herstellung racemischer Diphosphine der Formel (I) 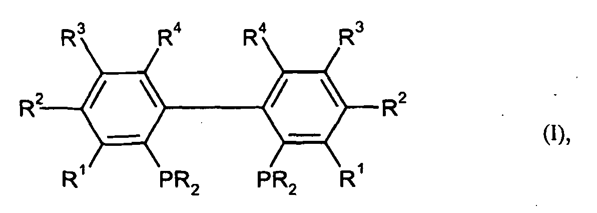 in der
- R: für C₆-C₁₄-Aryl oder 1 bis 3 Heteroatome aus der Gruppe Stickstoff, Sauerstoff und Schwefel enthaltendes C₄-C₁₃-Heteroaryl steht, wobei die Aryl- und Heteroarylreste

- R¹ bis R⁴: unabhängig voneinander jeweils für Wasserstoff, C₁-C₁₀-Alkyl, C₁-C₁₀-Alkoxy, F, Cl oder Br stehen,
das dadurch gekennzeichnet ist, dass man ein Phenol der Formel (II) 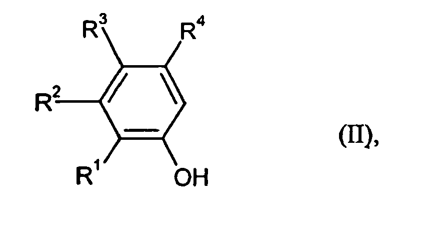 in der R¹ bis R⁴ die bei Formel (I) angegebene Bedeutung haben,
mit einer Base in das entsprechende Phenolat überführt, das Phenolat anschließend mit einem Dihalogenmethan zu einem Formaldehydacetal der Formel (III) umsetzt 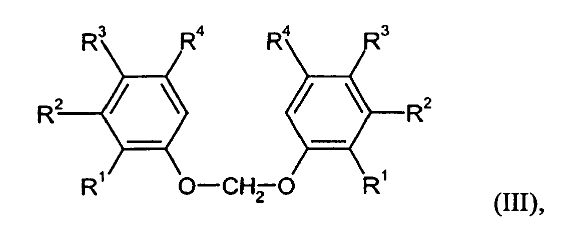 in der R¹ bis R⁴ die bei Formel (I) angegebene Bedeutung haben,
das Formaldehydacetal der Formel (III) innermolekular oxidativ kuppelt und so ein Cycloheptadien der Formel (IV) erhält, 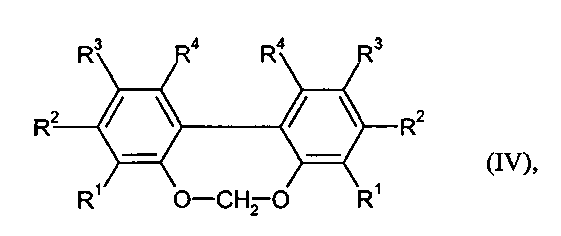 in der R¹ bis R⁴ die bei Formel (I) angegebene Bedeutung haben,
ein Cycloheptadien der Formel (IV) durch Behandlung mit einer Säure in ein Biphenyldiol der Formel (V) überführt 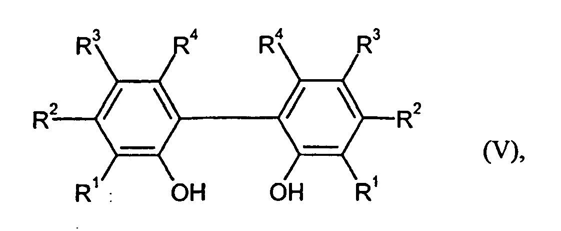 in der R¹ bis R⁴ die bei Formel (I) angegebene Bedeutung haben,
aus dem Biphenyldiol der Formel (V) das entsprechende Triflat herstellt und die Triflatverbindung mit einem sekundären Phosphan der Formel (VI)

HPR₂ (VI),

in der
R die bei Formel (I) angegebene Bedeutung hat,
unter Zusatz einer Base und in Gegenwart einer Palladium(0)-, Palladium(II)-, Nickel(0)- und/oder Ni(II)-Verbindung kuppelt und so eine Verbindung der Formel (I) erhält.

In den Formeln (I) bis (V) stehen R¹ und R² vorzugsweise für Wasserstoff und R³ und R⁴ vorzugsweise für C₁-C₅-Alkoxy, Fluor oder Chlor. In den Formeln (I) und (VI) steht R vorzugsweise für Phenyl, Furyl oder 2-N-C₁-C₆-Alkylpyrrolyl, die gegebenenfalls mit 1 bis 3 Substituenten aus der Gruppe Fluor, Chlor, C₁-C₅-Alkyl, C₁-C₆-Alkoxy und Trimethylsilyl substituiert sein können.

In den Formeln (I) bis (V) stehen R¹ und R² besonders bevorzugt für Wasserstoff, R³ besonders bevorzugt für Chlor, und R⁴ besonders bevorzugt für Methoxy oder Ethoxy.

In den Formeln (I) und (VI) steht R besonders bevorzugt für Phenyl, 2-Furyl, 2-N-Methylpyrrolyl, 3,5-Dimethylphenyl, 4-Fluorphenyl, 4-Tolyl oder 3,5-Dimethoxyphenyl.

Bei der Überführung des Phenols der Formel (II) in das entsprechende Phenolat kann man als Base z.B. ein Alkalihydrid, -hydroxid oder -carbonat einsetzen. Bevorzugt sind Natrium- und Kaliumhydrid. Die Base wird vorzugsweise in einer Menge von 0,9 bis 1,5 Äquivalenten pro Mol Phenol der Formel (II) eingesetzt. Man kann dabei in Gegenwart eines Lösungsmittels arbeiten, z.B. in Gegenwart eines dipolar-aprotischen Lösungsmittels wie Dimethylformamid oder eines Ethers wie Diethylether, Tetrahydrofuran, Dioxan oder Methyl-tert.-butyl-ether.

Geeignete Reaktionstemperaturen, insbesondere beim Einsatz von Alkalihydriden als Base, sind z.B. solche im Bereich -20 bis +60°C. Es ist vorteilhaft, diese Stufe unter einer Schutzgasatmosphäre durchzuführen. Man kann z.B. so verfahren, dass man die Base zusammen mit dem Lösungsmittel vorlegt und das Phenol der Formel (II), gelöst im gleichen Lösungsmittel zudosiert.

Das erhaltene Phenolat muss nicht isoliert werden. Insbesondere wenn man mit stöchiometrischen Mengen Alkalihydrid als Base gearbeitet hat, kann man das nach Umsetzung mit der Base vorliegende Reaktionsgemisch direkt weiter verwenden.

In die Umsetzung mit dem Phenolat kann man, bezogen auf ein Mol ursprünglich eingesetztes Phenol der Formel (II), z.B. 0,4 bis 0,7 Mole Dihalogenmethan einsetzen. Geeignete Reaktionstemperaturen sind beispielsweise solche von 0 bis 80°C, insbesondere solche von 10 bis 60°C. Die Reaktionszeit für die Umsetzung mit dem Dihalogenmethan kann z.B. 8 bis 40 Stunden betragen. Als Dihalogenmethan kommt z.B. Dichlor-, Dibrom- und Diiodmethan in Frage. Diiodmethan ist bevorzugt.

Das dann vorliegende Reaktionsgemisch kann man beispielsweise aufarbeiten, indem man es nach Zugabe von Wasser mit einem wenig polaren oder unpolaren organischen Lösungsmittel extrahiert und aus dem Extrakt das Lösungsmittel entfernt. Den verbleibenden Rückstand kann man gewünschtenfalls weiter reinigen, beispielsweise indem man ihn in einem Ether, in Methanol oder in Acetonitril bei erhöhter Temperatur löst, das Unlösliche verwirft und durch Kristallisation das hergestellte Formaldehydacetal der Formel (III) in gereinigter Form erhält.

Die innermolekulare oxidative Kupplung zur Herstellung eines Cycloheptadiens der Formel (IV) kann beispielsweise so durchgeführt werden, dass man dem Formaldehydacetal der Formel (III) zunächst eine lithiumorganische Verbindung und nach Beendigung deren Reaktion ein Oxidationsmittel hinzufügt. Beispielsweise kann man zu einer Lösung des Formaldehydacetals, beispielsweise in einem Ether, Butyllithium gelöst in beispielsweise einem Kohlenwasserstoff, bei -30 bis +40°C zugeben und durch Nachrühren bei einer Temperatur in diesem Bereich abreagieren lassen. Pro Mol Formaldehydacetal kann man z.B. 2,0 bis 2,2 Mole lithiumorganische Verbindung einsetzen. Im allgemeinen ist die Reaktion nach 5 bis 30 Stunden beendet. Dann kann man das Oxidationsmittel zugeben, beispielsweise eine Cu(II)-, Fe(III)-, Mn(III)- oder Ce(IV)-Verbindung. Die oxidative Kupplung ist auch enzymatisch, z.B. mit einer Peroxidase durchführbar. Die Zugabe des Oxidationsmittels erfolgt z.B. bei -70 bis -30°C und anschließender Erwärmung auf eine Temperatur von z.B. unter 50°C. Bezogen auf 1 Mol eingesetztes Formaldehydacetal der Formel (III) kann man z.B. 2,0 bis 2,5 Äquivalente eines Oxidationsmittels verwenden. Es ist vorteilhaft, das Reaktionsgemisch abschließend noch nachzurühren, z.B. für 1 bis 5 Stunden.

Es ist auch möglich, die oxidative Kupplung direkt aus dem Formaldehydacetal der Formel (III) nach den hier beschriebenen Methoden durchzuführen, ohne dieses zuvor in das Li-Salz zu überführen.

Es ist vorteilhaft, zumindest die Umsetzung mit der lithiumorganischen Verbindung unter einer Schutzgasatmosphäre durchzuführen.

Die Behandlung mit einer Säure zur Überführung eines Cycloheptadiens der Formel (IV) in ein Biphenyldiol der Formel (V) kann man beispielsweise mit einer starken Mineralsäure wie Salzsäure oder Schwefelsäure durchführen. Beispielsweise kann man 5 bis 15 Äquivalente Säure pro Mol Cycloheptadien der Formel (IV) einsetzen. Man arbeitet zweckmäßigerweise in Gegenwart eines Lösungsmittels, beispielsweise in Gegenwart eines Alkohols. Die Behandlung mit der Säure kann beispielsweise in einem Zeitraum von 5 bis 50 Stunden bei Temperaturen von 50 bis 100°C durchgeführt werden. Die Aufarbeitung des Reaktionsgemisches kann beispielsweise analog der oben bei der Herstellung von Formaldehydacetalen der Formel (III) beschriebenen erfolgen.

Die Herstellung der Triflatverbindung (=Trifluormethansulfonsäureester) aus dem Biphenyldiol der Formel (V) kann beispielsweise erfolgen, indem man das Biphenyldiol der Formel (V) in einem Lösungsmittel suspendiert, z.B. in einem aromatischen Kohlenwasserstoff, ein tertiäres Amin, z.B. Pyridin, zufügt und anschließend z.B. Trifluormethansulfonsäureanhydrid oder -chlorid, gegebenenfalls gelöst in einem Lösungsmittel, z.B. in einem aromatischen Kohlenwasserstoff, zudosiert und nachrührt. Das Zudosieren und Nachrühren kann z.B. bei 0 bis 60°C erfolgen. Es bildet sich dabei eine Suspension. Pro Mol Biphenyldiol der Formel (V) kann man z.B. 2 bis 3 Mole eines tertiären Amins und 2 bis 2,2 Mole Trifluormethansulfonsäureanhydrid oder -chlorid einsetzen.

Zur Aufarbeitung kann man das Reaktionsgemisch beispielsweise mit Wasser und wässriger Kochsalzlösung waschen, die verbleibende organische Phase trocknen und das Lösungsmittel, gegebenenfalls durch Abziehen im Vakuum, entfernen. Das so erhältliche Produkt ist rein genug für die Umsetzung mit sekundären Phosphanen. Gewünschtcnfalls kann man es weiter reinigen, z.B. durch (Flash)-Säulenchromatographie

Für die Umsetzung der Triflatverbindung mit einem sekundären Phosphan der Formel (VI) kann man als Base z.B. ein tertiäres Amin, etwa ein Trialkylamin verwenden, das drei gleiche oder verschiedene C₁-C₆-Alkylgruppen enthält. Arylalkylamine, DABCO, sogenannte Protonenschwämme - z.B. 1,8-Bis-(dimethylamino)-naphthalin - und Hydrogencarbonate wie Natriumhydrogencarbonat sind auch möglich. Bevorzugt setzt man Triethylamin oder Ethyl-diisopropyl-amin ein. Die Einsatzmenge der Base, bezogen auf ein Mol der Triflatverbindung, kann beispielsweise 2 bis 3 Mol betragen.

Als Palladium(0)- oder Nickel(0)-Verbindungen kommen beispielsweise Komplexe der Formeln (VIIa) und (VIIb) infrage,

Pd(PR'₃)₄ (VIIa)

Ni(PR'₃)₄ ) (VIIb

in denen
- R': jeweils für C₁-C₁₀-Alkyl oder C₆-C₁₄-Aryl steht, wobei Aryl gegebenenfalls durch Halogen und/oder C₁-C₆-Alkyl substituiert sein kann,
und wobei R' vorzugsweise für Phenyl steht.

Als Palladium(0)-Verbindung kommt auch Pd₂(dba)₃ in Frage, wobei dba für Dibenzylidenaceton steht. Das Pd₂(dba)₃ kann gegebenenfalls noch ein koordiniertes Lösungsmittelmolekül enthalten, z.B. CHCl₃.

Man kann auch eine Palladium(0)-Verbindung der Formel (VIIc) einsetzen

Pd(L₂) (VIIc),

in der
- L: für R'₂P-(CH₂)ₙ-PR'₂, Diphenylphosphinoferrocenyl oder 2,2'-Bis-(diphenylphosphinomethyl)-1,1'-binaphthyl steht, wobei R' die oben angegebene Bedeutung hat und n für 1, 2, 3 oder 4 steht.

Bevorzugte Verbindungen der Formel (VIIc) sind solche, bei denen L für R'₂P-(CH₂)ₙ-PR'₂ steht, wobei R' für Phenyl und n für 2, 3 oder 4 stehen.

Als Palladium(II)-Verbindung kommt z.B. Pd(CH₃COO)₂ in Frage, als Nickel(II)-Verbindung z.B. NiCl₂, das gegebenenfalls noch 1 bis 2 koordinierte PR'₃-Moleküle enthält (R' hat hier die gleiche Bedeutung wie bei den Formeln (VIIa) und (VIIb)).

Vorzugsweise gelangen Palladium(0)-Verbindungen der Formeln (VIIa) und (VIIc) sowie Pd₂(dba)₃ zum Einsatz. Diese Verbindungen kann man gewünschtenfalls auch in situ herstellen, beispielsweise indem man Palladiumdiacetat in einem Lösungsmittel vorlegt und den Liganden in der stöchiometrisch erforderlichen Menge oder im Überschuss von bis zu beispielsweise 150 % der stöchiometrisch erforderlichen Menge zufügt.

Die Einsatzmenge der Palladium- und/oder Nickelverbindungen, bezogen auf 1 Mol Triflatverbindung, kann beispielsweise 0,001 bis 0,1 Mol betragen.

Die Umsetzung der Triflatverbindung mit einem sekundären Phosphan der Formel (VI) kann man z.B. so durchführen, dass man die Palladium(0)-, Palladium(II)-, Nickel(0)- und/oder Nickel(II)-Verbindung in einem dipolar-aprotischen Lösungsmittel vorlegt oder in einem dipolar-aprotischen Lösungsmittel in situ herstellt und dann mit dem sekundären Phosphan der Formel (VI), der Base, der Triflatverbindung und gegebenenfalls weiterem Lösungsmittel zusammenbringt. Man kann auch wie zuvor beschrieben ein Gemisch herstellen, das die Palladium-und/oder Nickelverbindung enthält, dieses Gemisch zu vorgelegter Triflatverbindung hinzufügen und dann Base, sekundäres Phosphan der Formel (VI) und gegebenenfalls weiteres Lösungsmittel hinzufügen. Die Herstellung des Palladium- und/oder Nickelverbindungen enthaltenden Gemisches kann z.B. bei -10 bis +40°C erfolgen, die Umsetzung mit der Triflatverbindung z.B. bei 20 bis 160°C. Die Umsetzung der Triflatverbindung kann z.B. Reaktionszeiten im Bereich von 5 bis 200 Stunden beanspruchen.

Die Isolierung und Reinigung der so hergestellten Diphosphinverbindung der Formel (I) kann z.B. erfolgen, indem man zunächst bei erhöhter Temperatur im Vakuum das Lösungsmittel abzieht, den Rückstand mit Toluol aufnimmt, dieses Gemisch über eine Silica-Säule laufen lässt, die das hergestellte Diphosphin enthaltende Fraktion nimmt, daraus das Toluol abzieht, den Rückstand in Dimethylformamid löst und durch Überschichten mit Methanol oder Dialkylether das hergestellte Diphosphin kristallisiert.

Die vorliegende Erfindung betrifft weiterhin ein Verfahren zur Herstellung von enantiomerenreinen Diphosphinen der Formel (VIII) 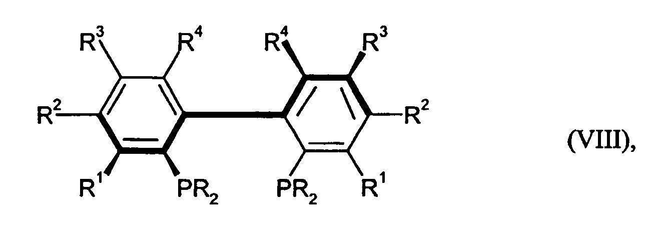 in der die verwendeten Symbole die bei Formel (I) angegebene Bedeutung haben und einer Formel, die Formel (VIII) analog ist, jedoch das andere Enantiomer darstellt.

Diese Herstellung wird erfindungsgemäß durchgeführt wie die oben beschriebene Herstellung der racemischen Diphosphine der Formel (I) und ist zusätzlich dadurch gekennzeichnet, dass man das Biphenyldiol der Formel (V) einer Racematspaltung unterwirft. Die Racematspaltung kann beispielsweise durch Kristallisation unter Verwendung eines Hilfsreagenzes oder durch chirale Chromatographie z.B. nach der SMB-Methode erfolgen. Geeignete Hilfsreagenzien für die Racematspaltung durch Kristallisation sind z.B. Weinsäure- und Cinchonin-Derivate.

Bevorzugt verwendet man hierfür (-)-O,O'-Dibenzoyl-L-weinsäure oder enantiomerenreines N-Benzylcinchonidiniumchlorid. Pro Mol Biphenyldiol kann beispielsweise 0,5 bis 1 Mol Hilfsreagenz eingesetzt werden.

Die Racematspaltung kann man z.B. so durchführen, dass man das racemische Biphenyldiol der Formel (V) zusammen mit dem Hilfsreagenz in einem geeigneten Lösungsmittel, z.B. einem C₁-C₄-Alkylalkohol oder Acetonitril für einige Stunden am Rückfluss erhitzt, nachrührt, den vorliegenden Niederschlag abfiltriert und in einem mit Wasser nicht mischbaren Lösungsmittel, z.B. einem Chloralkan, einem aromatischen Kohlenwasserstoff oder Essigsäureethylester aufnimmt, mit einer Säure, z.B. einer verdünnten Mineralsäure, wäscht, die organische Phase abtrennt, die wässrige Phase mit einem mit Wasser nicht mischbaren Lösungsmittel extrahiert und aus den vereinigten organischen Phasen das Lösungsmittel abzieht.

Die sonstige Herstellung von enantiomerenreinen Diphosphinen der Formel (VIII) erfolgt dann wie oben für die Herstellung racemischer Diphosphine beschrieben.

Die vorliegende Erfindung betrifft weiterhin enantiomerenreine Diphosphine der Formel (IX) 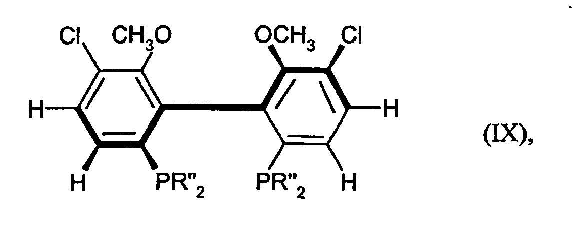 in der die Reste R" jeweils gleich sind und für 2-Furyl, 2-N-Methylpyrrolyl, 4-Fluorphenyl, 3,5-Di-methoxyphenyl oder 3,5-Dimethylphenyl stehen und einer Formel, die Formel (IX) analog ist, jedoch das andere Enantiomer darstellt.

Die vorliegende Erfindung betrifft weiterhin Cycloheptadienverbindungen der Formel (IV), racemische und enantiomerenreine Biphenyldiole der Formel (V) und die aus den Biphenyldiolen der Formel (V) zugänglichen entsprechenden racemischen und enantiomerenreinen Triflatverbindungen, bei denen jeweils R¹ und R² für H stehen, R³ jeweils für Chlor steht und R⁴ jeweils für Methoxy steht.

Die erfindungsgemäß hergestellten racemischen Diphosphine der Formel (I) und die neuen enantiomerenreinen (+)- und (-)-Diphoshine der Formel (VIII) eignen sich als Liganden zur Herstellung von Katalysatoren, vorzugsweise von Katalysatoren für Hydrierungen. Die enantiomerenreinen (+)- und (-)-Diphosphine der Formel (VIII) eignen sich insbesondere als Liganden zur Herstellung von Hydrierkatalysatoren für enantioselektive Hydrierungen.

Die genannten Liganden können, um zu Hydrierkatalysatoren zu gelangen, mit Metallen, auch in Form von Metallionen oder Metallkomplexen von Elementen der VIII. Nebengruppen des Periodensystems kombiniert werden. Ruthenium, Iridium und Rhodium sind dabei bevorzugt. Die Kombination Ligand-Metall kann dabei separat oder in situ im Reaktionsgemisch für die Hydrierung vorgenommen werden. Dabei kann man pro Mol Metall z.B. 0,5 bis 10 Mol, vorzugsweise 1 bis 5 Mol der genannten Liganden einsetzen.

Die racemischen Diphosphine der Formel (I) sind z.B. mit Vorteil einsetzbar als Liganden für Palladiumkatalysatoren, die in Aminierungsreaktionen Verwendung finden. Durch Palladiumkomplexe katalysierte Aminierungen sind zahlreiche Zwischenprodukte für Pharma- und Pflanzenschutzwirkstoffe zugänglich. Bisher ist bekannt, für solche Aminierungen Binaphthylphosphorverbindungen als Liganden einzusetzen.

Die vorliegende Erfindung betrifft schließlich auch noch Katalysatoren, die ein Metall, ein Metallion oder einen Metallkomplex eines Elements der VIII. Nebengruppe des Periodensystems und wenigstens ein Diphosphin der Formel (IX) enthalten. Vorzugsweise enthalten diese Katalysatoren unabhängig voneinander Ruthenium, Iridium oder Rhodium und pro Mol Metall, Metallion oder Metalkomplex 0,5 bis 10 Mol eines Diphosphins der Formel (IX).

Beim erfindungsgemäßen Verfahren zur Herstellung von racemischen Diphosphinen der Formel (I) ist vorteilhaft, dass eine breite Palette an unterschiedlichen Liganden direkt aus einer Vorstufe (= einer Verbindung der Formel (VI)) zugänglich ist. So ist es leicht möglich, unterschiedliche, für ein spezielles Katalysatorproblem maßgeschneiderte Liganden mit unterschiedlichen elektronischen und sterischen Verhältnissen herzustellen.

Beim erfindungsgemäßen Verfahren zur Herstellung von enantiomerenreinen Diphosphinen der Formel (VIII) ist vorteilhaft, dass die Phosphinreste erst nach der Racematspaltung eingeführt werden. Dadurch kann man die aufwendige Racematspaltung für diverse Diphosphine auf einer gemeinsamen Vorstufe durchführen und erst danach ein breites Spektrum individueller Diphosphine herstellen. Separate Racematspaltungen für einzelne Diphosphine können dadurch vermieden werden.

Die erfindungsgemäßen enantiomerenreinen Diphosphine der Formel (IX) haben den Vorteil, dass man aus ihnen Katalysatoren herstellen kann, die hinsichtlich des nach ihrer Anwendung erzielbaren Enantiomerenüberschusses anderen Katalysatoren in verschiedenen Reaktionen überlegen sind. Rutheniumkatalysatoren mit erfindungsgemäßen enantiomerenreinen Liganden sind z.B. vorteilhaft bei der enantioselektiven Hydrierung von heteroaromatischen Ketonen und Itaconsäurederivaten.

Die erfindungsgemäßen Cycloheptadienverbindungen, Biphenyldiole und Triflatverbindungen sind neue Zwischenprodukte zur Herstellung von neuen Diphosphinen, aus denen sich Katalysatoren mit überlegenen Eigenschaften herstellen lassen.

### Beispiele

### Beispiel 1

### Herstellung von Formaldehyd-[bis-(4-chlor-3-methoxyphenyl)-acetal]

### (IUPAC: Bis(4-chlor-3-methoxyphen-1-oxy)methan)

Zu einer Suspension von 16,0 g Natriumhydrid (95 %ig) in 300 ml Dimethylformamid wurde unter Argon bei 0°C langsam eine Lösung von 100 g 4-Chlor-3-methoxyphenol in 250 ml Dimethylformamid getropft. Nach beendeter Zugabe wurde noch 1 Stunde bei 40°C nachgerührt, wobei eine klare, gelbe Lösung entstand. Bei Raumtemperatur wurde nun langsam eine Lösung aus 88,1 g Diiodmethan in 100 ml Dimethylformamid zugetropft. Die Lösung wurde 15 Stunden bei Raumtemperatur gerührt, wobei allmählich eine orange-rote Suspension entstand. Anschließend wurde noch 3 Stunden bei 50°C gerührt. Man gab 400 ml Wasser zu und extrahierte dreimal mit je 150 ml Methylenchlorid. Die vereinigten organischen Phasen wurden dreimal mit je 100 ml gesättigter wässriger Kochsalzlösung gewaschen, die organischen Phasen über Natriumsulfat getrocknet und das Lösungsmittel bei 50°C im Vakuum entfernt, wobei ein orange-brauner fester Rückstand anfiel. Dieser wurde in 250 ml Methyl-tert.-butyl-ether in der Siedehitze gelöst, von vorhandenem, öligen Rückstand abdekantiert und eingeengt bis Niederschlagsbildung eintrat. Man ließ erst bei Raumtemperatur, dann zur Vervollständigung der Fällung bei +4°C kristallisieren. Der Niederschlag wurde abfiltriert und vorsichtig mit Methyl.-tert.-butylether/Petrolether 1:1 gewaschen. Die Mutterlauge wurde eingeengt und erneut kristallisieren gelassen.
Ausbeute: 85,2 g (82 % der Theorie)
Schmelzpunkt: 110°C

Durch Entfernung des DMF vor der Aufarbeitung mit Dichlormethan/Wasser kann die chemische Ausbeute von 82 auf 90 % gesteigert werden. Die vorhandenen, stark gefärbten Oxidationsprodukte des Phenols lassen sich durch Flashsäulenchromatographie über Kieselgel mit Dichlormethan als Eluent abtrennen.

| | |
|---|---|
| ¹H-NMR (CDCl₃): δ = | 3,87 (s, 6H, CH₃); 5,68 (s, 2H, CH₂); 6,65-6,70 (m, 4H, H_{arom.}); 7,27 (d, ³J_{H-H} = 8,1 Hz, 2H, H_{arom.}) |
| ¹³C-NMR (CDCl₃): δ = | 56,2 (CH₃); 91,4 (CH₂); 102,0 (C_{arom.}); 108,2 (C_{arom.}); 116,2 (C_{arom., ipso}); 130,4(C_{arom.}); 155,7 (C_{arom, ipso});156,5 (C_{arom., ipso}); |

### Beispiel 2

Herstellung von 2,10-Dichloro-1,11-dimethoxy-5,7-dioxa-dibenzo[a,c]cycloheptadien

Zu einer Lösung aus 60 g Formaldehyd-[bis-(4-chlor-3-methoxyphenyl)-acetal] (IUPAC: Bis(4-chlor-3-methoxyphen-1-oxy)methan) in 100 ml Tetrahydrofuran wurden bei 0°C unter Argon 239 ml einer 1,6 molaren Lösung von Butyllithium in Hexan langsam zugetropft. Nach beendeter Zugabe rührte man 15 Stunden bei Raumtemperatur, wobei eine gelbe Suspension entstand. Die Reaktionsmischung wurde dann auf -50°C gekühlt und 51,3 g wasserfreies Kupfer(II)-chlorid zugegeben. Dann ließ sich die Lösung innerhalb von 5 Stunden auf Raumtemperatur erwärmen und gab dann 300 ml Wasser und 200 ml Methylenchlorid zu. Es wurde mit 50 ml 2n wässriger Salzsäure neutralisiert und dann der entstandene weiß-graue Niederschlag durch Zugabe von 300 ml 25 %iger wässriger Ammoniaklösung wieder in Lösung gebracht. Die Methylenchlorid-Phase wurde abgetrennt und die tief dunkelblaue wässrige Phase noch 5 mal mit je 100 ml Methylenchlorid extrahiert. Anschließend wurde die organische Phase noch einige Male mit insgesamt 400 ml gesättigter wässriger Ammoniumchloridlösung gewaschen, bis diese nur noch schwach blau gefärbt war. Die organische Phase wurde etwas eingeengt, über Natriumsulfat getrocknet und das Lösungsmittel vollständig entfernt, wobei ein brauner Feststoff anfiel, der mit 100 ml siedendem Methyl-tert.-butylether behandelt wurde. Die gebildete Lösung wurde vom öligen Rückstand abdekantiert und man ließ die Lösung bei +4°C kristallisieren. Der ausgefallene Feststoff wurde abfiltriert, danach aus dem Filtrat im Vakuum das Lösungsmittel entfernt und der sich dabei bildende Rückstand einer säulenchromatographischen Reinigung mit Kieselgel 60 und mit Toluol als Eluent unterworfen. Das gelbe Eluat wurde im Vakuum vom Lösungsmittel befreit und der Rückstand erneut in 40 ml siedendem Methyl-tert.-butylether gelöst und bei +4°C kristallisieren gelassen.

### Ausbeute: 45,4 g (77 % der Theorie)

Durch vorherige Trocknung des Kupfer(II)-chlorids über P₄O₁₀ bei 140°C und Verwendung einer äquimolaren Mischung aus n-Butyllithium und N,N,N,N-Tetramethyl(ethylendiamin) (TMEDA) kann eine sehr selektive Aromatenkupplung zum Wunschprodukt erzielt werden (Roh-NMR zeigt nur ein Produkt). Als vorteilhaft hat sich weiterhin das Entfernen des THF vor der Aufarbeitung und eine saure Aufarbeitung mit 4 N HCl erwiesen.
Die Ausbeute lässt sich durch diese Maßnahmen auf 95 % der Theorie steigern.

**Schmelzpunkt: 130°C**

| | |
|---|---|
| ¹H-NMR (CDCI₃): δ = | 3,60 (s, 6H, OCH₃); 5,46 (s, 2H, OCH₂O); 6,94 (d, ³J = 8,7 Hz, 2H, Hₐᵣₒₘ); 7,43 (d, ³J = 8,7 2H, H_{arom.}) |
| ¹³C-NMR (CDCI₃): δ = | 61,2 (CH₃); 102,3 (CH₂); 117,0 (C_{arom.}); 124,1 (C_{arom., ipso}); 124,9 (C_{arom., ipso}); 130,8 (Cₐᵣₒₘ.); 152,1 (C_{arom., ipso}); 154,4 (C_{arom., ipso}); |

### Beispiel 3

### Herstellung von 5,5'-Dichloro-6,6'-dimethoxy-biphenyl-2,2'-diol

Zu einer Suspension aus 1,76 g 2,10-Dichloro-1,11-dimethoxy-5,7-dioxa-dibenzo-[a,c]cycloheptadien in 25 ml Ethanol wurden 4 ml konzentrierte wässrige Salzsäure gegeben. Anschließend wurde unter Argon 21 Stunden am Rückfluss erhitzt, wobei der Reaktionsverlauf mittels Dünnschichtchromatographie mit Methylenchlorid als Laufmittel kontrolliert wurde. Die entstandene klare, gelbe Lösung wurde mit 30 ml Wasser versetzt, zweimal mit je 50 ml Methylenchlorid extrahiert und zweimal mit je 50 ml gesättigter wässriger Kochsalz-Lösung gewaschen. Die organische Phase wurde über Natriumsulfat getrocknet, filtriert und das Lösungsmittel im Vakuum entfernt. Der Rückstand wurde vorsichtig mit kaltem Chloroform verrieben, dieses wieder abdekantiert und verworfen.

### Ausbeute: 1,54 g (91 % der Theorie)

Durch Zugabe von 1,5 Äquivalenten Ethylenglykol, bezogen auf die molare Menge des eingesetzten Acetals, kann die Ausbeute auf 99 % der Theorie gesteigert werden.

**Schmelzpunkt: 110°C**

| | |
|---|---|
| ¹H-NMR (CDCI₃): δ = | 3,66 (s, 6H, OCH₃); 5,29 (s, 2H, OH); 6,84 (d, ³J_{H-H} = 9,0 Hz, 2H, H_{arom.}); 7,37 (d, ³J_{H-H} = 8,7 2H, Hₐᵣₒₘ.) |
| ¹³C-NMR (CDCl₃): δ = | 61,1 (CH₃); 114,4 (Cₐᵣₒₘ.); 115,2 (C_{arom. ipso}); 119,1 (C_{arom., ipso}); 131,5 (C_{arom.}); 153,6 (C_{arom., ipso}); 153,8 (C_{arom., ipso}); |

### Beispiel 4

### Herstellung von (+)-5,5'-DichIoro-6,6'-dimethoxy-biphenyI-2,2'-dioI

Eine Suspension aus 22,9 g 5,5'-Dichloro-6,6'-dimethoxy-biphenyl-2,2'-diol und 16,0 g N-Benzylcinchonidiniumchlorid in 110 ml Acetonitril wurden 4 Stunden zum Rückfluss erhitzt und anschließend 15 Stunden bei Raumtemperatur gerührt. Die enstandene Fällung wurde abfiltriert, mit wenig Acetonitril gewaschen und im Vakuum getrocknet. Der Rückstand wurde in 250 ml Essigester aufgenommen und zweimal mit je 50 ml wässriger 2n Salzsäure ausgeschüttelt. Die organische Phase wurde abgetrennt und die wässrige Phase noch zweimal mit je 50 ml Essigester extrahiert. Die vereinigten organischen Phasen wurden viermal mit je 100 ml gesättigter wässriger Kochsalzlösung gewaschen, über Natriumsulfat getrocknet, filtriert und das Lösungsmittel im Vakuum entfernt.

Ausbeute: 6,77 g (30 % der Theorie)
Enantiomerenreinheit : 98,4 e.e.
Die Kontrolle der Enantiomerenreinheit erfolgt durch analytische HPLC.
Als Laufinittel wird n-Heptan/Isopropanol 80:20 verwendet
[α]_{D} = + 23,6 (c = 1,5; CHCl₃)

Eine anschließende Umkristallisation aus Chloroform ergab ein Produkt mit einer Enantiomerenreinheit von über 99,9 % e.e.

### Beispiel 5

### Herstellung von (5,5'-Dichloro-6,6'-dimethoxy-biphenyl-2,2'-diyl)-bis-trifluormethansulfonsäureester

3,4 g (+)-5,5'-Dichloro-6,6'-dimethoxy-biphenyl-2,2'-diol wurden in 40 ml Toluol suspendiert und 2,5 g Pyridin zugegeben, wobei innerhalb von 10 min. eine klare, leicht braun gefärbte Lösung entstand. Zu dieser Lösung wurde bei Raumtemperatur eine Lösung aus 6,9 g Trifluormethansulfonsäureanhydrid in 5 ml Toluol getropft. Es bildete sich schnell ein flockiger Niederschlag. man rührte 3 Stunden bei 45°C, wobei eine orange-farbene Suspension entstand. Diese Suspension wurde zweimal mit je 20 ml Wasser und danach zweimal mit je 30 ml gesättigter wässriger Kochsalzlösung gewaschen. Die organische Phase wurde über Natriumsulfat getrocknet, filtriert und das Lösungsmittel bei 50°C im Vakuum entfernt, wobei ein orange-farbenes Öl anfiel. Dieses war rein genug um direkt weiter eingesetzt zu werden. Es kann gegebenenfalls durch Flashsäulenchromatographie an Kieselgel mit Toluol als Eluent weiter gereinigt werden.

**Ausbeute an Öl: 5,7 g (92 % der Theorie)**

| | |
|---|---|
| ¹H-NMR (CDC1₃): δ = | 3,77 (s, 6H, OCH₃); 5,29 (s, 2H, OH); 7,18 (d, ³J_{H-H} = 9,0 Hz, 2H, H_{arom.}); 7,58 (d, ³J_{H-H} = 9,0 Hz, 2H, H_{arom.}) |
| ¹³C-NMR (CDCl₃): δ = | 61,5 (CH₃); 117,2 (C_{arom.}); 118,3 (q, ¹J(C, F) = 320 Hz, CF₃); 121,1 (C_{arom. ipso}); 127,8 (C_{arom., ipso}); 132,3 (C_{arom.}); 145,9 (C_{arom., ipso}); 155,9 (C_{arom., ipso}); |
| ¹⁹F-NMR (CDCI₃): δ = | 74,9 (s, CF₃) |

### Beispiel 6

### Herstellung von (5,5'-Dichloro-6,6'-dimethoxy-biphenyl-2,2'-diyl)-bis-(diphenylphosphin)

220 mg Pd(PPh₃)₄ wurden mit 75 mg Diphenylphosphinopropan unter Argon in 10 ml Dimethylsulfoxid gegeben und 3 Stunden bei Raumtemperatur gerührt, wobei eine orangefarbene Suspension entstand. Zu dieser Suspension wurden 0,99 g Diphenylphosphin, 0,85 g N,N-Diisopropylethylamin, 1,00 g (5,5'-Dichloro-6,6'-dimethoxy-biphenyl-2,2'-diyl)-bis-trifluormethansulfonsäureester und weitere 10 ml Dimethylsulfoxid gegeben und die klare, gelbe Lösung anschließend 79 Stunden bei 100°C gerührt. Das Lösungsmittel wurde nach beendeter Reaktion im Vakuum bei 100°C entfernt, der Rückstand mit 10 ml Methanol versetzt und bei -25°C kristallisieren gelassen. Der entstandene, feine Niederschlag wurde abfiltriert und mit Methanol gewaschen.

Ausbeute: 0,70 g (62 % der Theorie).

Die NMR-Daten waren identisch mit den in der EP-A 749 973 angegebenen.

### Beispiel 7

### Herstellung von (5,5'-Dichloro-6,6'-dimethoxy-biphenyl-2,2'-diyl)-bis-(bis-2-furylphosphin)

100 mg Pd₂(Dibenzylidenaceton)₃ · CHCl₃ wurden zusammen mit 80 mg Diphenylphosphinopropan unter Argon in 10 ml Dimethylformamid suspendiert und 2 Stunden bei Raumtemperatur gerührt, wobei eine klare, orangefarbene Lösung entstand. Diese Lösung wurde mit einer Kanüle in ein Schlenk-Gefäß überführt, in dem 3,40 g (5,5'-Dichloro-6,6'-dimethoxy-biphenyl-2,2'-diyl)-bis-trifluormethansulfonsäureester vorgelegt waren. Zu dieser Lösung wurden weitere 10 ml Dimethylformamid, 1,56 g Triethylamin und 1,00 g Di-2-furylphosphin gegeben und anschließend 72 Stunden bei 100°C gerührt, wobei nach 22 Stunden weitere 1,03 g Bis-2-furylphosphin zugegeben wurden. Das Lösungsmittel wurde nach beendeter Reaktion bei 100°C im Vakuum entfernt, der Rückstand eine Stunde mit 20 ml Diethylether im Ultraschallbad behandelt und die etherische Lösung vom braunen, öligen Rückstand abdekantiert. Der Ether wurde im Vakuum entfernt, der verbleibende Feststoff in 2 ml Dimethylformamid aufgenommen, vorsichtig mit 10 ml Methanol überschichtet und bei +4°C kristallisieren gelassen.
Ausbeute: 0,85 g (24 % der Theorie).

**Schmelzpunkt: 150°C**

| | |
|---|---|
| ¹H-NMR (CD₃CN): δ = | 3,28 (s, 6H, OCH₃); 6,37 (m, 2H, Hₐᵣₒₘ); 6,44 (d, ³J_{H-H} = 3,3 Hz, 2H, H_{arom.}); 6,48 (m, 2H, H_{arom.}); 6,65 (d, ³J_{H-H} = 3,3 Hz, 2H, H_{arom.}); 7,44 (dt, ³J_{H-H} = 8,1 Hz, ³J_{H-H} = 8,1 Hz, , ³J_{H-P} = 1,5 Hz, 2H, H_{arom.}); 7,52 (d, , ³J_{H-H} = 8,4 Hz, 2H, H_{arom.}); 7,64 (d, ³J_{H-H} = 1,8 Hz, 2H, Hₐᵣₒₘ); 7,77 (d, ³J_{H-H} = 1,8 Hz, 2H, Hₐᵣₒₘ.); |
| ¹³C-NMR (CDCl₃): δ = | 60,4 (CH₃); 110,6 (Cₐᵣₒₘ.); 110,8 (Cₐᵣₒₘ); 121,4 (Cₐᵣₒₘ.); 121,8 (C_{arom.}); 129,2 (C_{arom., ipso}); 130,2 (C_{arom.}); 130,6 (C_{arom.}); 134,4 (C_{arom., ipso}); 136,7 (C_{arom., ipso}); 147,4 (C_{arom.}); 149,4 (C_{arom. ipso}); 150,2 (C_{arom., ipso}); 154,3 (C_{arom., ipso}); |
| ³¹P-NMR (CDCl₃) δ = | -59,14 |

Eine Wiederholung dieses Beispiels unter Verwendung von N,N-Dimethylacetamid anstelle von Dimethylformamid ergab bei einer Reaktionszeit von 12 Stunden das gleiche Produkt.

### Beispiel 8

### Herstellung von (5,5'-Dichloro-6,6'-dimethoxy-biphenyl-2,2'-diyl)-bis-(bis-p-fluorphenyl-phosphin)

100 mg Pd₂(Dibenzylidenaceton CHCl₃) wurden zusammen mit 80 mg Diphenylphosphinopropan unter Argon in 10 ml Dimethylformamid suspendiert und 2 Stunden bei Raumtemperatur gerührt, wobei eine klare, orangefarbene Lösung entstand. Diese Lösung wurde mit einer Kanüle in ein Schlenk-Gefäß überführt, in dem 3,28 g (5,5'-Dichloro-6,6'-dimethoxy-biphenyl-2,2'-diyl)-bis-trifluonmethansulfonsäureester vorgelegt waren. Zu dieser Lösung wurden weitere 10 ml Dimethylformamid, 1,50 g Triethylamin und 1,25 g Bis-(p-fluorphenyl)-phosphin gegeben und anschließend 72 Stunden bei 100°C gerührt, wobei nach 23 Stunden weitere 1,75 g Bis-(p-fluorphenyl)phosphin zugegeben wurden. Das Lösungsmittel wurde nach beendeter Reaktion im Vakuum bei 100°C entfernt, der Rückstand eine Stunde mit 10 ml Diethylether im Ultraschallbad behandelt und die Lösung vom braunen, öligen Rückstand abdekantiert. Der Ether wurde im Vakuum entfernt, der Rückstand in 2 ml Dimethylformamid aufgenommen, vorsichtig mit 10 ml Methanol überschichtet und bei +4°C kristallisieren gelassen.
Ausbeute: 0,80 g (20 % der Theorie).

**Schmelzpunkt: 139°C**

| | |
|---|---|
| ¹H-NMR (CD₃CN): δ= | 3,35 (s, 6H, OCH₃); 6,88-7,13 (m, 14H, H_{arom.}); 7,16-7,27 (m, 4H, H_{arom.}); 7,46 (d, ³J_{H-H} = 8,1 Hz, 2H, H_{arom.}) |
| ¹³C-NAMR (CDCl₃): δ= | 60,2 (CH₃); 115,5 (Cₐᵣₒₘ); 115,8 (C_{arom.}); 128,7 (C_{arom.,ipso}); 130,2 (Cₐᵣₒₘ.); 130,7 (C_{arom.}); 131,7 (C_{arom.,ipso}); 132,7 (C_{arom.,ipso}); 134,6 (C_{arom.}); 136,2 (C_{arom.}); 137,8 (C_{arom., ipso}); 154,3 (C_{arom., ipso}); 161,4 (C_{arom.,ipso}); 161,9 (C_{arom. ipso}); 164,7 (C_{arom., ipso}); 165,2 (C_{arom., ipso}); |
| ³¹P-NMR (CDCl₃) δ = | -16,01 |
| ¹⁹F-NMR (CDCl₃) δ = | 113,6 (s, Ar-F); -112,3 (s, Ar-F) |

### Beispiel 9

### Herstellung von (5,5'-Dichloro-6,6'-dimethoxy-biphenyl-2,2'-diyl)-bis-(bis-3,5-dimethylphenyl-phosphin)

100 mg Pd₂(Dibenzylidenaceton)₃ CHCl₃ wurden zusammen mit 80 mg Diphenylphosphinopropan unter Argon in 10 ml Dimethylformamid suspendiert und 1 Stunde bei Raumtemperatur gerührt, wobei eine klare, orangefarbene Lösung entstand. Diese Lösung wurde mit einer Kanüle in ein Schlenk-Gefäß überführt, in dem 2,70 g (5,5'-Dichloro-6,6'-dimethoxy-biphenyl-2,2'-diyl)-bis-trifluormethansulfonsäureester vorgelegt waren. Zu dieser Lösung wurden weitere 10 ml Dimethylformamid, 1,60 g N,N-Diisopropylethylamin und 2,43 g Bis-(3,5-dimethylphenyl)-phosphin gegeben und anschließend insgesamt 115 Stunden bei 100°C gerührt, wobei nach 75 Stunden weitere 0,25 g und nach 100 Stunden weitere 0,32 g Bis-(3,5-dimethylphenyl)-phosphin zugegeben wurden. Das Dimethylformamid wurde nach beendeter Reaktion im Vakuum bei 100°C entfernt und der Rückstand einer Flashsäulenchromatographie über Kieselgel 60 mit Toluol als Eluent unterworfen. Das Lösungsmittel wurde im Vakuum entfernt, der Rückstand in 2 ml Dimethylformamid aufgenommen, vorsichtig mit 8 ml Methanol überschichtet und bei +4°C kristallisieren gelassen. Nach Abtrennen der 1. Fällungsfraktion wurde das Filtrat bei 100°C auf 1 ml eingeengt und erneut nach Überschichten mit Methanol kristallisieren gelassen.

Ausbeute: 1,50 g (42 % der Theorie).

**Schmelzpunkt: 225°C**

| | |
|---|---|
| ¹H-NMR (CD₃CN): δ = | 2,14 (s, 12H, Ar-CH₃); 2,22 (s, 12H, Ar-CH₃); 3,27 (s, 6H, OCH₃); 6,80-6,90 (m, 12H, H_{arom.}); 6,99 (d, ³J_{H-H} = 9,0 Hz, 2H, H_{arom.}); 7,31 (d, ³J_{H-H} = 9,0 Hz, 2H, H_{arom.}); |
| ³¹P-NMR (CDC1₃): δ = | -13,73 |

### Beispiel 10-1

### Herstellung von (5,5'-Dichloro-6,6'-dimethoxy-biphenyl-2,2'-diyl)-bis-(bis-2-(-N-methylpyrrolyl)-phosphin)

### a) Herstellung von Di(N-methylpyrrolyl)-ethylphosphinit

Zu einer Lösung von 38,61 g Methylpyrrol in 200 ml Diethylether wurden bei 0°C zunächst 297,5 ml einer 1,6 molaren Lösung von n-Butyllithium in Hexan und dann 35,0 g Dichlorethylphosphinit zugetropft. Anschließend wurde die Reaktionslösung über Nacht bei Raumtemperatur gerührt und dann das Lösungsmittel im Vakuum abgezogen. Der verbleibende Rückstand wurde in 200 ml Petrolether aufgenommen, die unlöslichen Lithiumsalze abfiltriert und aus dem Filtrat das Lösungsmittel abgezogen. Der dabei anfallende Rückstand wurde fraktioniert destilliert.
Ausbeute: 14,14 g (25 % der Theorie).
Siedepunkt: 125 - 130°C bei 0,17 Torr.
³¹P-NMR-Verschiebung: 76,54 ppm in CDCl₃

### b) Herstellung von Di-2-(N-methylpyrrolyl)-phosphin

Zu einer Suspension von 0,75 g Lithiumaluminiumhydrid in 20 ml Tetrahydrofuran wurden bei -70°C 2,15 g Trimethylchlorsilan getropft und die Suspension dann 2 Stunden bei Raumtemperatur gerührt. Anschließend wurde eine Lösung von 2,15 g Di-(N-methylpyrrolyl)-ethylphosphinit in 10 ml Tetrahydrofuran hinzugetropft. Die Reaktionsmischung wurde 20 Stunden bei Raumtemperatur gerührt. Danach wurde die Reaktionsmischung mit 1 g Wasser hydrolysiert (bis zur Beendigung der Wasserstoffentwicklung), das Lösungsmittel im Vakuum abgezogen und der verbleibende Rückstand in Petrolether aufgenommen. Nach Abfiltrierung der unlöslichen Aluminium- und Lithiumsalze wurde aus dem Filtrat das Lösungsmittel abgezogen und der dabei anfallende Rückstand im Vakuum getrocknet.
Ausbeute: 30 % der Theorie.
³¹P-NMR-Verschiebung: -111,36 ppm in C₆D₆

### c) Synthese der eingangs genannten Verbindung

Es wurde verfahren wie in Beispiel 6 beschrieben, wobei anstelle von Diphenylphosphin Di-2-(N-methylpyrrolyl)-phosphin eingesetzt wurde.

| | |
|---|---|
| ¹H-NMR (CDCl₃): δ = | 3,04 (s, 6H); δ = 3,26 (s, 6H); δ = 3,66 (s, 6H); δ = 5,98-6,01 (m, 2H); δ = 6,65 (t, 2H); δ = 6,07-6,1 (m, 2H); δ = 6,17 (t, 2H); δ = 6,65 (t, 2H); δ = 6,84-6,87 (m, 2H); δ = 6,88-6,93 (m, 2H); δ = 7,3 (d, 2H); |
| ³¹P-NMR (CDC1₃): δ = | -58,75 |

### Beispiel 10-2

### Herstellung von (5,5'-Dichloro-6,6'-dimethoxy-biphenyl-2,2'-diyl)-bis-(bis-3,5-dimethoxyphenyl)-phosphin)

In einem ausgeheizten Schlenk-Gefäß mit Teflonrührkern wurden 26,4 mg [Pd₂(dba)₃] · CHCl₃ und 1,1 mg Diphenylphosphinopropan in 3 ml Dimethylacetamid suspendiert und 30 min. bei Raumtemperatur gerührt, wobei die Lösung klar wurde und eine rote Farbe annahm. Diese Lösung wurde mit einer Kanüle in ein Schlenk-Gefäß überführt, in dem 0,95 g (5,5'-Dichloro-6,6'-dimethoxy-biphenyl-2,2'-diyl)-bis-trifluormethansulfonsäureester und 1,1 g Bis-(3,5-dimethoxyphenyl)-phosphan in 10 ml Dimethylacetamid vorgelegt waren. Anschließend wurden 0,64 g Diisopropylethylamin hinzugefügt und auf 80°C erwärmt. Nach 72 Stunden wurde das Lösungsmittel im Vakuum entfernt, der Rückstand in 2 ml Toluol aufgenommen und mittels Flash-Chromatographie an Kieselgel mit Toluol als Laufmittel gereinigt. Das sich ergebende gelbliche Öl wurde in Diethylether aufgenommen und der Ether dann im Vakuum entfernt. So wurden 0,78 g des Produkts als hellgelber voluminöser Stoff erhalten.
Ausbeute: 53 % der Theorie
³¹P-NMR-(CDCl₃) : δ = -10,61
MS (SIMS): m/z (%) = 890.9 (9, M⁺), 753.0 (5, M⁺-C₈H₁₀O₂), 584.9 [100, M⁺-P(C₈H₁₀O₂)₂], 448.9 [2, M⁺-P(C₈H₁₀O₂₎₂-C₈H₁₀O₂], 384.9 3, 337.0 5, 280.9 {19, M⁺-[P(C₈H₁₀O₂)₂]₂} 220.9 (28), 206.9 (33), 146.9 (70).

### Beispiel 11

### Herstellung des Katalysators (5,5'-Dichloro-6,6'-dimethoxy-biphenyl-2,2'-diyl)-bis-(di-3,5-dimethylphenylphosphino)-bis-(3,3,3-trifluoraceto)-ruthenium

0,0431 g (Cylooctadien)Ru(η³-methallyl)₂ und 0,1033 g (5,5'-Dichloro-6,6'-dimethoxy-biphenyl-2,2'-diyl)-bis-(bis-3,5-dimethylphenylphosphin) wurden in 5 ml Methylenchlorid gelöst, 5 ml Methanol zugegeben und dann unter Rühren 21,5 ul Trifluoressigsäure zugefügt. Nachdem man 24 Stunden bei Raumtemperatur gerührt hatte, wurde das Lösungsmittel im Vakuum entfernt und der orangefarbene Rückstand 2 Stunden im Vakuum getrocknet.

### Beispiel 12

### Herstellung des Katalysators (5,5'-Dichloro-6,6'-dimethoxy-biphenyl-2,2'-diyl)-bis-(bis-4-fluorphenylphosphino)-bis-(3,3,3-trifluoraceto)-ruthenium

Die Herstellung erfolgte wie in Beispiel 11 beschrieben, wobei anstelle von (5,5'-Dichloro-6,6'-dimethoxy-biphenyl-2,2'-diyl)-bis-(bis-3,5-dimethylphenylphosphin) jetzt das entsprechende Bis-4-fluorphenyl-phosphin eingesetzt wurde.

### Beispiel 13-1

### Herstellung des Katalysator (5,5'-Dichloro-6,6'-dimethoxy-biphenyl-2,2'-diyl)-bis-(di-2-furylphosphino)-bis-(3,3,3-trifluoraceto)-ruthenium

Die Herstellung erfolgte wie in Beispiel 11 beschrieben, wobei anstelle von (5,5' Dichloro-6,6'-dimethoxy-biphenyl-2,2'-diyl)-bis-(bis-3,5-dimethylphenylphosphin) jetzt das entsprechende Bis-2-furylphosphin eingesetzt wurde.

### Beispiel 13-2

### Herstellung des Katalysator (5,5'-Dichloro-6,6'-dimethoxy-biphenyl-2,2'-diyl)-bis-(3,5-dimethoxyphenyl-phosphino)-bis-(3,3,3-trifluoraceto)-ruthenium

Zu einer Lösung von 155 mg (Cyclooctadien)Ru(η³-methallyl)₂ in CH₂Cl₂ wurden 432 mg des Produktes aus Beispiel 10-2 gegeben und 1 Stunde bei Raumtemperatur gerührt. Dann wurde das Lösungsmittel im Vakuum entfernt. Das Produkt fiel als dunkelgrüner Feststoff in quantitativer Ausbeute an.
³¹P-NMR-(d⁴-MeOH) : δ = 64,22
MS (SIMS): m/z (%) = 1143.2, 991.2 [12, M⁺-(CO₂CF₃)₂], 585.3 [M⁺-Ru(CO₂CF₃)₂-P(CgH₁₀O₂)₂], 147.1 (19), 132.9 (28), 73.1 (100).

### Beispiele 14 bis 20

### Hydrierungen mit Katalysatoren aus den Beispielen 11 bis 13.

20 µmol Katalysator wurden in 5 ml Methanol gelöst, 2,0 ml Itaconsäure-dimethylester und 0,100 g Diglyme (GC-Standard) zugefügt und in einem Glasautoklaven (1 bar H₂) oder Stahlautoklaven (70 bar H₂) mit Wasserstoff umgesetzt. Nach beendeter Reaktionszeit wurde, gegebenenfalls nach Druckentspannung, zum Entfernen von gelöstem Wasserstoff Vakuum angelegt und die Katalysator/Produkt-Lösung anschließend gaschromatographisch untersucht. Die Ergebnisse sind in Tabelle 1 zusammengefasst.

**Tabelle 1**

| **Beispiel** | **Katalysator aus Beispiel** | **Temp. (°C)** | **Druck (bar)** | **Reaktionszeit (h)** | **Umsatz (%)** | **Ausbeute (%)** | **TOF / h⁻¹** | **ee (%)** | **Konfiguration** |
|---|---|---|---|---|---|---|---|---|---|
| 14 | 13-1 | 23 | 70 | 15 | 99,9 | 100 | 6,6 | 65.5 | (S) |
| 15 | 13-1 | 50 | 1 | 1 | 17,0 | 12,2 | 12,3 | 92,5 | (S) |
| 16 | 12 | 22 | 1 | 1 | 70 | 71 | 70 | 94,7 | (S) |
| 17 | 12 | 22 | 1 | 0,7 | 35 | 34 | 49,8 | 96,2 | (S) |
| 18 | 12 | 50 | 1 | 0,5 | 100 | 100 | 198 | 95,5 | (S) |
| 19 | 11 | 22 | 1 | 1 | 100 | 100 | 22 | 94,7 | (S) |
| 20 | 11 | 50 | 1 | 0,5 | 100 | 100 | >200 | 96,9 | (S) |

### Beispiele 21 bis 24

### Hydrierungen mit in situ-Katalysator-Systemen

20 µmol (Norbornadien)₂ RhPF₆ wurden mit jeweils 20 mmol der Liganden aus den Beispielen 7 bis 9 in 5 ml eines Lösungsmittels gelöst. Beim Liganden aus Beispiel 7 war das Lösungsmittel Methanol, bei den Liganden aus den Beispielen 8 und 9 war das Lösungsmittel ein 1:1-Gemisch aus Methylenchlorid und Methanol. Das erhaltene Gemisch wurde 1 Stunde bei 40°C gerührt, dann die Lösungsmittel im Vakuum entfernt und dann 5 ml Methanol zugegeben. Es wurden 2,0 mmol Itaconsäuredimethylester und 0,100 g Diglyme (GC-Standard) zugefügt und in einem Glasautoklaven (1 bar H₂) oder Stahlautoklaven (70 bar H₂) mit Wasserstoff umgesetzt. Nach beendeter Reaktionszeit wurde, gegebenenfalls nach Druckentspannung, zum Entfernen von gelöstem Wasserstoff Vakuum angelegt und die Katalysator/ProduktLösung anschließend, gaschromatographisch untersucht. Die Ergebnisse sind in Tabelle 2 zusammengefasst.

**Tabelle 2**

| **Beispiel** | **ligand** | **Temp. (°C)** | **Druck (bar)** | **Reaktionszeit (h)** | **Umsatz (%)** | **Ausbeute (%)** | **TOF / h⁻¹** | **ee (%)** | **Konfiguration** |
|---|---|---|---|---|---|---|---|---|---|
| 21 | aus Beispiel 7 | 24 | 1 | 1 | 95 | 100 | 120 | 31,4 | (S) |
| 22 | aus Beispiel 7 | 22 | 70 | 0,5 | 97 | 95 | 193 | 14,3 | (S) |
| 23 | aus Beispiel 8 | 22 | 1 | 1 | 12 | 16 | 22 | 26 | (S) |
| 24a) | aus Beispiel 9 | 22 | 1 | 1 | 100 | 100 | 98,5 | 78,3 | (S) |
| 24b) | aus Beispiel 9 | 22 | 100 | 0,5 | 100 | 100 | 196 | 58,5 | (S) |
| 24c) | aus Beispiel 9 | 50 | 100 | 0,5 | 100 | 100 | >200 | 74,2 | (S) |

### Beispiele 25 bis 28

### Hydrierungen mit dem Katalysator aus Beispiel 13-2

In einem ausgeheizten und mit Argon beschickten Glasautoklaven (im Beispiel 27 wurde ein Stahlautoklav verwendet) wurde eine Lösung von 0,32 g Dimethylitakonat, 0,024 g des Katalysators aus Beispiel 13-2, 0,1 g Diglyme und 5 ml Methanol gegeben und dann mit 1 bar (in Beispiel 27 70 bar) Wasserstoff beschickt. Anschließend wurde bei der in Tabelle 3 angegebenen Temperatur 30 min intensiv gerührt. Danach wurde nach Entfernung des Wasserstoffs eine Probe zur Bestimmung des Umsatzes (GC) entnommen, das verbleibende Gemisch einer Flash-Destillation unterworfen und im Destillat der Enantiomerenüberschuss bestimmt.

Im Beispiel 28 wurden 0,64 g Dimethylitakonat eingesetzt. Einzelheiten sind aus Tabelle 3 ersichtlich.

**Tabelle 3**

| **Beispiel** | **Temp. (°C)** | **Druck (bar)** | **Umsatz (%)** | **TOF / h⁻¹** | **ee (%)** |
|---|---|---|---|---|---|
| 25 | 22 | 1 | 99,9 | 138 | 92,4 |
| 26 | 40 | 1 | 99,7 | >200 | 92,4 |
| 27 | 22 | 70 | 99,7 | >200 | 55,6 |
| 28 | 22 | 1 | 38,3 | 136 | 91,2 |

## Patentansprüche

1. Verfahren zur Herstellung enantiomerenreiner Diphosphine der Formel (VIII) 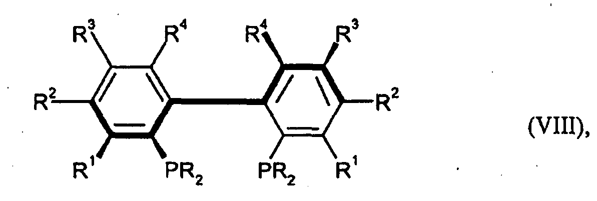 und einer Formel, die Formel (VIII) analog ist, jedoch das andere Enantiomer darstellt,
in der
R für 2-Furyl, 2-N-Methylpyrrolyl, 3,5-Dimethylphenyl, 4-Fluorphenyl, 4-Tolyl oder 3,5-Dimethoxyphenyl, und
R¹ bis R⁴ unabhängig voneinander jeweils für Wasserstoff, C₁-C₁₀-Alkyl, C₁-C₁₀-Alkoxy, F, Cl oder Br stehen,
**dadurch gekennzeichnet ist, dass** man ein Phenol der Formel (II) 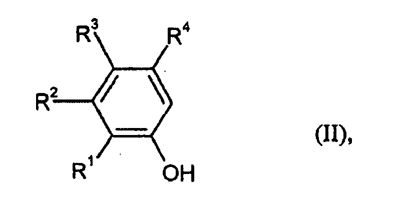 in der R¹ bis R⁴ die bei Formel (VIII) angegebene Bedeutung haben,
mit einer Base in das entsprechende Phenolat überführt, das Phenolat anschließend mit Dihalogenmethan zu einem Formaldehydacetal der Formel (III) umsetzt 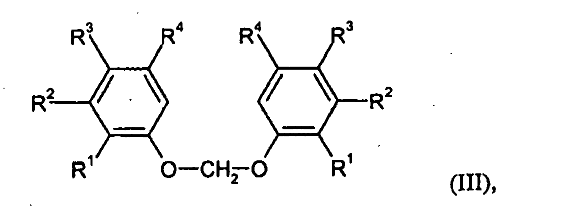 in der R¹ bis R⁴ die bei Formel (VIII) angegebene Bedeutung haben,
das Formaldehydacetal der Formel (III) innermolekular oxidativ kuppelt und so ein Cycloheptadien der Formel (IV) erhält, 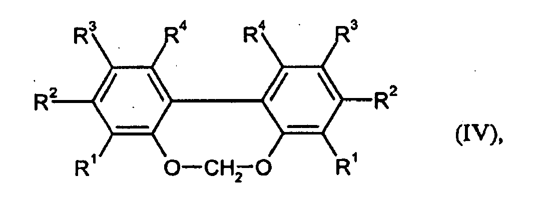 in der R¹ bis R⁴ die bei Formel (VIII) angegebene Bedeutung haben,
ein Cycloheptadien der Formel (IV) durch Behandlung mit einer Säure in ein Biphenyldiol der Formel (V) überführt 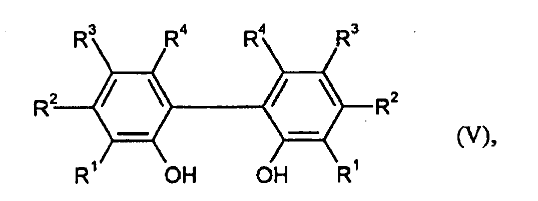 in der R¹ bis R⁴ die bei Formel (VIII) angegebene Bedeutung haben,
das Biphenyldiol der Formel (V) einer Racematspaltung unterwirft;
aus dem Biphenyldiol der Formel (V) das entsprechende Triflat herstellt und die Triflatverbindung mit einem sekundären Phosphan der Formel (VI)
HPR₂ (VI),
in der
R die bei Formel (VIII) angegebene Bedeutung hat,
unter Zusatz einer Base und in Gegenwart einer Palladium(0)-, Palladium(II)-, Nickel(0)- und/oder Ni(II)-Verbindung kuppelt und so eine Verbindung der Formel (I) erhält.

2. Enantiomerenreine Diphosphine der Formel (IX) 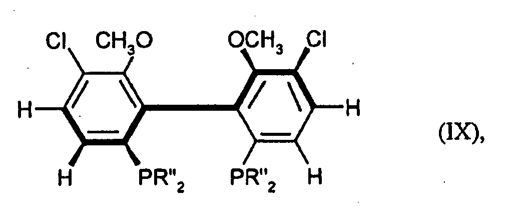 in der die Reste R" jeweils gleich sind und für 2-Furyl, 2-N-Methylpyrrolyl oder 4-Fluorphenyl stehen und einer Formel, die Formel (IX) analog ist, jedoch das andere Enantiomer darstellt.

3. Cycloheptadienverbindungen der Formel (IV) 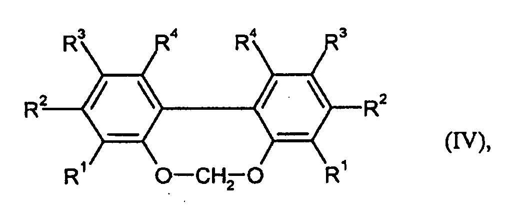 enantiomerenreine Biphenyldiole der Formel (V) 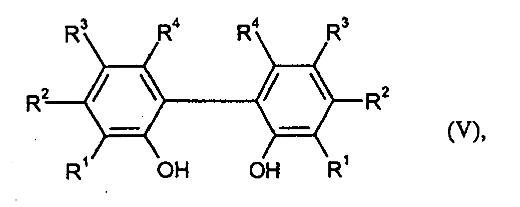 und die aus den Biphenyldiolen der Formel (V) zugänglichen entsprechenden enantiomerenreinen Triflatverbindungen, wobei in den Formeln (IV) und (V) und bei den aus den Biphenyldiolen der Formel (V) zugänglichen Triflatverbindungen, wobei R¹ und R² jeweils für H stehen, R³ jeweils für Chlor steht und R⁴ jeweils für Methoxy steht.

4. Verwendung von enantiomerenreinen Diphosphinen der Formel (IX) gemäß Anspruch 2 als Liganden zur Herstellung von Katalysatoren.

5. Katalysatoren, **dadurch gekennzeichnet, dass** sie ein Metall, Metallion, der Metallkomplex eines Elements der VIII. Nebengruppe des Periodensystems und wenigstens ein enantiomerenreines Diphosphin gemäß Anspruch 2 enthalten.

6. Verfahren nach Anspruch 1, **dadurch gekennzeichnet, dass** in den Formeln (I) bis (IV) R¹ und R² für Wasserstoff und R³ und R⁴ für C₁-C₄-Alkoxy, Fluor oder Chlor stehen.

7. Verfahren nach Anspruch 1, **dadurch gekennzeichnet, dass** man
das Phenol der Formel (II) mit einem Alkalihydrid, -hydroxid oder -carbonat bei -20 bis +60°C in das entsprechende Phenolat überführt,
bezogen auf 1 mol ursprünglich eingesetztes Phenol der Formel (II) 0,4 bis 0,7 mol Dihalogenmethan mit dem Phenolat umsetzt,
das Formaldehydacetal der Formel (III) nach Zugabe von Wasser mit einem wenig polaren oder unpolaren organischen Lösungsmittel aus dem Reaktionsgemisch isoliert,
zur innermolekularen oxidativen Kupplung zur Herstellung eines Cycloheptadiens der Formel (IV) zunächst eine lithiumorganische Verbindung bei -30 bis +40°C zufügt und dann eine Cu(II)-, Fe(III)-, Mn(III)- oder Ce(IV)-Verbindung bei -70 bis -30°C, oder diese Kupplung enzymatisch durchführt,
die Behandlung mit einer Säure zur Überführung des Cycloheptadiens der Formel (IV) in ein Biphenyldiol der Formel (V) mit 5 bis 15 Äquivalenten einer starken Mineralsäure durchführt,
zur Herstellung der Triflatverbindung das Biphenyldiol der Formel (V) in einem Lösungsmittel suspendiert und ein tertiäres Amin und anschließend Trifluormethansulfonsäureanhydrid oder -chlorid hinzugibt und
als Base für die Umsetzung der Triflatverbindung mit einem sekundären Phosphan ein tertiäres Amin und ein Hydrogencarbonat verwendet.

8. Katalysatoren nach Anspruch 5, **dadurch gekennzeichnet, dass** sie Ruthenium, Iridium oder Rhodium enthalten.

## Claims

1. Process for the preparation of enantiomerically pure diphosphines of the formula (VIII) 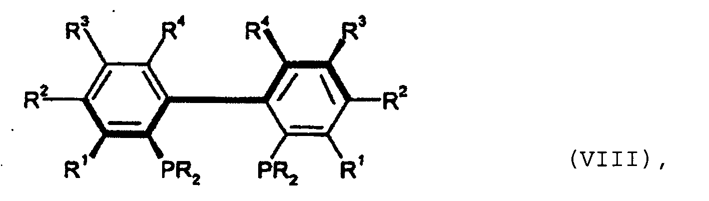 and of a formula which is analogous to formula (VIII), but represents the other enantiomer,
in which
R is 2-furyl, 2-N-methylpyrrolyl, 3,5-dimethylphenyl, 4-fluorophenyl, 4-tolyl or 3,5-dimethoxyphenyl, and
R¹ to R⁴, independently of one another, are each hydrogen, C₁-C₁₀-alkyl, C₁-C₁₀-alkoxy, F, Cl or Br,
**characterized in that** a phenol of the formula (II) 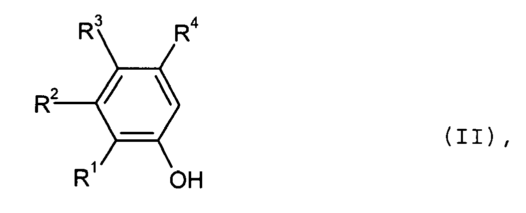 in which R¹ to R⁴ have the meanings given for formula (VIII),
is converted into the corresponding phenoxide using a base, the phenoxide is then reacted with dihalogenomethane to give a formaldehyde acetal of the formula (III) 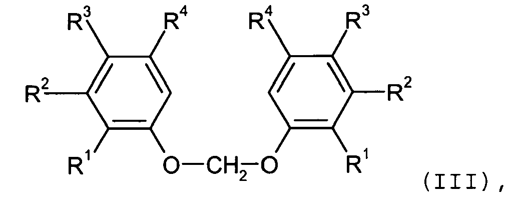 in which R¹ to R⁴ have the meanings given for formula (VIII),
the formaldehyde acetal of the formula (III) is intramolecularly oxidatively coupled giving a cycloheptadiene of the formula (IV), 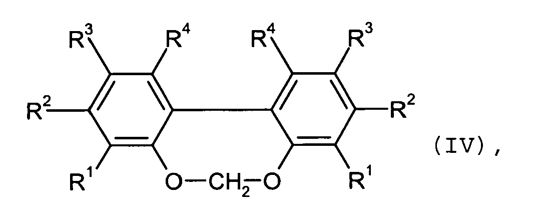 in which R¹ to R⁴ have the meanings given for formula (VIII),
a cycloheptadiene of the formula (IV) is converted by treatment with an acid into a biphenyldiol of the formula (V) 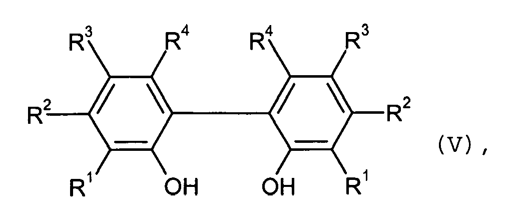 in which R¹ to R⁴ have the meanings given for formula (VIII),
the biphenyldiol of the formula (V) is subjected to racemate resolution;
the corresponding triflate is prepared from the biphenyldiol of the formula (V), and the triflate compound is coupled with a secondary phosphine of the formula (VI)
HPR₂ (VI),
in which
R has the meaning given for formula (VIII),
with the addition of a base and in the presence of a palladium(0), palladium(II), nickel(0) and/or Ni(II) compound, giving a compound of the formula (I).

2. Enantiomerically pure diphosphines of the formula (IX) 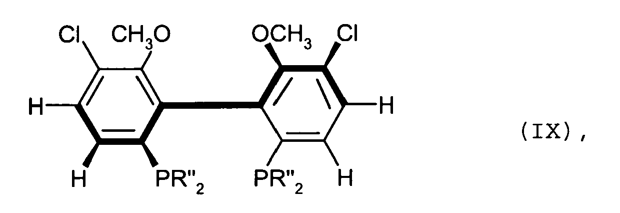 in which the radicals R'' are in each case identical and are 2-furyl, 2-N-methylpyrrolyl or 4-fluorophenyl, and of a formula which is analogous to formula (IX), but represents the other enantiomer.

3. Cycloheptadiene compounds of the formula (IV) 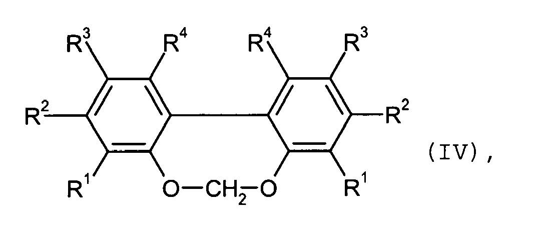 enantiomerically pure biphenyldiols of the formula (V) 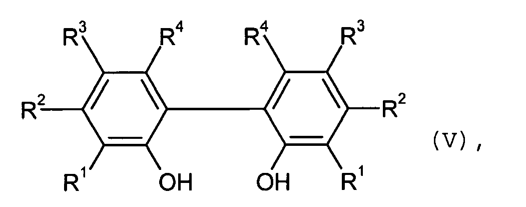 and the corresponding enantiomerically pure triflate compounds accessible from the biphenyldiols of the formula (V), where, in the formulae (IV) and (V) and in the triflate compounds accessible from the biphenyldiols of the formula (V), where R¹ and R² in each case are H, R³ in each case is chlorine and R⁴ in each case is methoxy.

4. Use of enantiomerically pure diphosphines of the formula (IX) according to Claim 2 as ligands for the preparation of catalysts.

5. Catalysts **characterized in that** they contain a metal, metal ion, the metal complex of an element from subgroup VIII of the Periodic Table of the Elements and at least one enantiomerically pure diphosphine according to Claim 2.

6. Process according to Claim 1, **characterized in that**, in the formulae (I) to (IV), R¹ and R² are hydrogen and R³ and R⁴ are C₁-C₄-alkoxy, fluorine or chlorine.

7. Process according to Claim 1, **characterized in that**
the phenol of the formula (II) is converted into the corresponding phenoxide using an alkali metal hydride, hydroxide or carbonate at -20 to +60°C,
based on 1 mol of phenol of the formula (II) originally used, 0.4 to 0.7 mol of dihalogenomethane is reacted with the phenoxide,
the formaldehyde acetal of the formula (III) is isolated from the reaction mixture following the addition of water using a virtually nonpolar or nonpolar organic solvent,
for the intramolecular oxidative coupling for the preparation of a cycloheptadiene of the formula (IV), firstly an organolithium compound is added at -30 to +40°C and then a Cu(II), Fe(III), Mn(III) or Ce(IV) compound at -70 to -30°C, or this coupling is carried out enzymatically,
the treatment with an acid to convert the cycloheptadiene of the formula (IV) into a biphenyldiol of the formula (V) is carried out with 5 to 15 equivalents of a strong mineral acid,
for the preparation of the triflate compound, the biphenyldiol of the formula (V) is suspended in a solvent, and a tertiary amine and then trifluoromethanesulphonic anhydride or trifluoromethanesulphonyl chloride are added and
the base used for the reaction of the triflate compound with a secondary phosphine is a tertiary amine and a hydrogencarbonate.

8. Catalysts according to Claim 5, **characterized in that** they contain ruthenium, iridium or rhodium.

## Revendications

1. Procédé pour la préparation de diphosphines sous forme d'énantiomères purs, de formule (VIII) 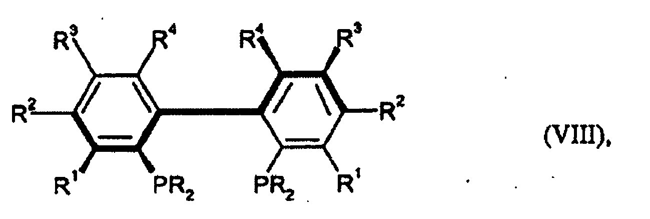 et d'une formule qui est analogue à la formule (VIII), mais représente l'autre énantiomère,
formule dans laquelle
R représente le groupe 2-furyle, 2-N-méthylpyrrolyle, 3,5-diméthylphényle, 4-fluorophényle, 4-tolyle ou 3,5-diméthoxyphényle, et
R¹ à R⁴ représentent chacun, indépendamment les uns des autres, un atome d'hydrogène, un groupe alkyle en C₁-C₁₀, alcoxy en C₁-C₁₀, F, Cl ou Br,
**caractérisé en ce qu'**on convertit un phénol de formule (II) 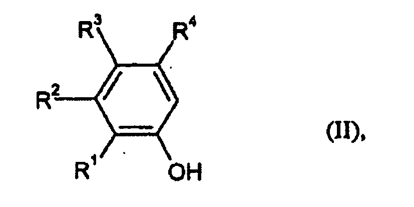 dans laquelle R¹ à R⁴ ont les significations données à propos de la formule (VIII),
à l'aide d'une base, en le phénolate correspondant, on fait ensuite réagir le phénolate avec un dihalogénométhane, pour aboutir à un formaldéhyde-acétal de formule (III) 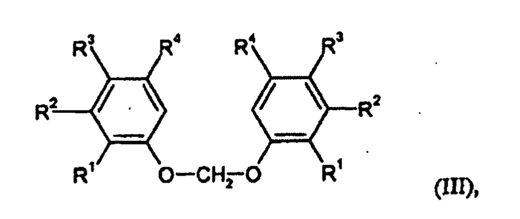 dans laquelle R¹ à R⁴ ont les significations données à propos de la formule (VIII),
on soumet le formaldéhyde-acétal de formule (III) à un couplage oxydant intramoléculaire et on obtient ainsi un cycloheptadiène de formule (IV), 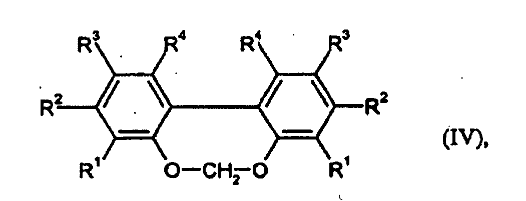 dans laquelle R¹ à R⁴ ont les significations données à propos de la formule (VIII),
on convertit un cyclopentadiène de formule (IV), par traitement par un acide, en un biphényldiol de formule (V) 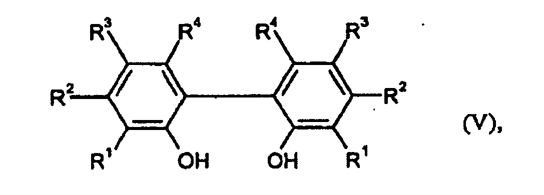 dans laquelle R¹ à R⁴ ont les significations données à propos de la formule (VIII),
on soumet le biphényldiol de formule (V) à un dédoublement de racémate ;
on prépare le triflate correspondant à partir du biphényldiol de formule (V) et on combine le triflate avec un phosphane secondaire de formule (VI)
HPR₂ (VI),
dans laquelle R a la signification donnée à propos de la formule (VIII),
avec addition d'une base et en présence d'un composé de palladium-(0), palladium-(II), nickel-(0) et/ou Ni-(II) et on obtient ainsi un composé de formule (I).

2. Diphosphines sous forme d'énantiomères purs, de formule (IX) 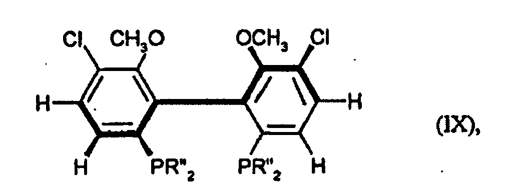 dans laquelle les radicaux R" sont identiques et représentent chacun un groupe 2-furyle, 2-N-méthylpyrrolyle ou 4-fluorophényle et, d'une formule qui est analogue à la formule (IX), mais représente l'autre énantiomère.

3. Composés cycloheptadiène de formule (IV) 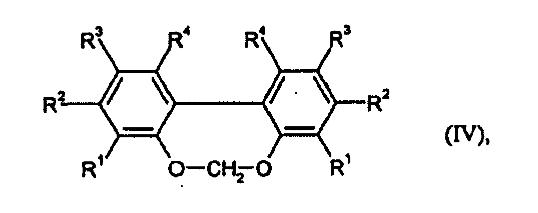 biphényldiols sous forme d'énantiomères purs de formule (V) 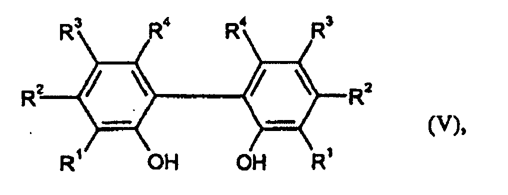 et les triflates correspondants, sous forme d'énantiomères purs, accessibles à partir des biphényldiols de formule (V), dans les formules (IV) et (V) et dans les triflates accessibles à partir des biphényldiols de formule (V) R¹ et R² représentant chacun H, R³ représentant chaque fois un atome de chlore et R⁴ représentant chaque fois le groupe méthoxy.

4. Utilisation de diphosphines sous forme d'énantiomères purs, de formule (IX) selon la revendication 2, en tant que ligands pour la préparation de catalyseurs.

5. Catalyseurs, **caractérisés en ce qu'**ils contiennent un métal, un ion métallique, le complexe métallique d'un élément du VIIIème groupe secondaire du système périodique et au moins une diphosphine sous forme d'énantiomère pur selon la revendication 1.

6. Procédé selon la revendication 1, **caractérisé en ce que** dans les formules (I) à (IV) R¹ et R² représentent un atome d'hydrogène et R³ et R⁴ représentent un groupe alcoxy en C₁-C₄, un atome de fluoro ou de chlore.

7. Procédé selon la revendication 1, **caractérisé en ce que**
on convertit le phénol de formule (II), à l'aide d'un hydrure, hydroxyde ou carbonate de métal alcalin, à une température de -20 à +60 °C, en le phénolate correspondant,
on fait réagir avec le phénolate 0,4 à 0,7 mole de dihalogénométhane, pour 1 mole de phénol de formule (II) initialement utilisé,
on isole à partir du mélange réactionnel, après addition d'eau avec un solvant organique peu polaire ou non polaire, le formaldéhyde-acétal de formule (III),
pour le couplage oxydant intramoléculaire pour la préparation d'un cyclopentadiène de formule (IV), on ajoute d'abord un composé organolithié, à une température de -30 à +40 °C, et ensuite un composé de Cu-(II), Fe-(III), Mn- (III) ou Ce- (IV) à une température de -70 à -30 °C ou on effectue ce couplage par voie enzymatique,
on effectue le traitement par un acide pour la conversion du cycloheptadiène de formule (IV) en un biphényldiol de formule (V), avec 5 à 15 équivalents d'un acide minéral fort,
pour la préparation du triflate, on met le biphényldiol de formule (V) en suspension dans un solvant et on y ajoute une amine tertiaire et ensuite de l'anhydride trifluorométhaneulfonique ou du chlorure de trifluorométhanesulfonyle et
pour la réaction du triflate avec un phosphane secondaire, on utilise comme base une amine tertiaire et un hydrogénocarbonate.

8. Catalyseurs selon la revendication 5, **caractérisés en ce qu'**ils contiennent du ruthénium, de l'iridium ou du rhodium.
